# FASCICULE DE BREVET EUROPEEN

(11) **EP 3 332 728 B1**
(45) Date de publication et mention de la délivrance du brevet: **09.10.2019**
(21) Numéro de dépôt: 17205921.4
(22) Date de dépôt: 07.12.2017
(51) Int. Cl.: A61B 18/24

(54) **DISPOSITIF DE TRAITEMENT ENDOVEINEUX AVEC ELEMENT FILAIRE SOUPLE GUIDE**
VORRICHTUNG ZUR ENDOVENÖSEN BEHANDLUNG MIT EINER MITTELS BIEGSAMER FÜHRUNG GEFÜHRTEN FASER
ENDOVENOUS TREATMENT DEVICE HAVING A FIBER ELEMENT GUIDED BY A SOFT GUIDE

(30) Priorité: 08.12.2016 FR 1662137
(43) Date de publication de la demande: 13.06.2018
(73) Titulaire: LSO MEDICAL, 59120 Loos (FR)
(72) Inventeur: ROCHON, Philippe, 59182 Loffre (FR)
(74) Mandataire: Matkowska, Franck

(56) Documents cités:
- US-A1- 2003 236 517
- US-A1- 2005 203 497
- US-A1- 2015 250 489

## Description

### Domaine technique

La présente invention concerne le domaine du traitement endoveineux, par délivrance dans la veine d'une dose de traitement, au moyen d'un élément filaire souple. La dose de traitement peut, de manière non limitative et non exhaustive, être une dose d'énergie, délivrée par exemple sous la forme d'un rayonnement électromagnétique, au moyen d'ondes sonores ou ultrasonores, d'ondes radiofréquences, ou être une dose d'énergie thermique délivrée par rayonnement et/ou par contact, ou une dose d'un produit permettant un traitement de la veine. L'élément filaire souple peut être creux ou plein, et peut notamment, de manière non limitative et non exhaustive, être une fibre optique, un élément filaire de type câble ou une sonde souple ou une canule souple.

### Art antérieur

Dans le domaine du traitement endoveineux, il est connu de traiter une veine en délivrant dans la veine des doses de traitement au moyen d'un élément filaire souple, qui est inséré longitudinalement dans la veine, et dont le mouvement de retrait doit être contrôlé en cours de traitement. Plus particulièrement, dans le domaine de la thérapie par laser endoveineux, plus connue sous l'acronyme EVLT, il est usuel de traiter une veine au moyen d'un laser endoveineux (par exemple occlusion des varices saphènes par laser endoveineux), dont l'élément filaire souple est une fibre optique utilisée pour émettre un rayonnement électromagnétique dans la veine. Pour d'autres types de traitement endoveineux, l'élément filaire souple peut, de manière non limitative et non exhaustive, également être un câble ou une sonde souple ou une canule souple.

Des exemples de dispositif de traitement endoveineux sont décrits par exemple dans les publications suivantes :
US2003/0236517, US 2005/0131400,
US2008/0097224, US2008/0097408, US 6 814 727.

De manière usuelle, le retrait de l'élément filaire souple inséré dans la veine, et par exemple de la fibre optique, peut être commandé en cours de traitement par le praticien au moyen d'un système de retrait motorisé permettant de tirer sur la partie extrême proximale (la plus éloignée du corps du patient) de l'élément filaire souple en contrôlant la vitesse de retrait. Ce retrait peut selon le cas être effectué de manière continue ou pas à pas.

Il est intéressant de pouvoir mettre en oeuvre un élément filaire ayant une longueur importante, de manière à pouvoir facilement déporter, en dehors du champ opératoire stérile, le système de retrait motorisé et le système de délivrance de doses de traitement, tel que par exemple la source laser dans le cas d'un laser endoveineux. La partie extrême distale (la plus proche du corps du patient) de l'élément filaire doit pouvoir être introduite dans la veine, et l'élément filaire doit être suffisamment souple pour pouvoir remonter la partie extrême distale de l'élément filaire dans la veine, jusqu'au site à traiter, la veine pouvant avoir un chemin plus ou moins tortueux. En conséquence, à ce jour un retrait déporté, exercé en dehors du champ stérile, nécessite de mettre sous tension l'élément filaire souple, afin de transmettre efficacement, à la partie distale de l'élément filaire, la traction exercée sur la partie extrême proximale de l'élément filaire souple. En conclusion, avec les solutions existantes, on est en pratique amené à tendre l'élément filaire entre son point d'insertion et le système de retrait.

Cette mise sous tension de l'élément filaire souple occasionne plusieurs inconvénients.

Elle augmente d'une manière générale les risques de retraits accidentels et non contrôlés en dehors de la veine de l'élément filaire souple durant la procédure de traitement. En particulier le moindre mouvement du patient ou le moindre choc sur l'élément filaire souple peut de manière préjudiciable provoquer des retraits accidentels et non contrôlés de l'élément filaire souple. Le poids de l'élément filaire, surtout lorsqu'il présente une longueur importante, et/ou le poids du cathéter d'introduction utilisé pour introduire l'élément filaire souple dans la veine peuvent de manière préjudiciable occasionner des retraits intempestifs et non contrôlés de l'élément filaire durant la procédure de traitement.

La mise en tension de l'élément filaire oblige également à positionner le système de retrait dans l'axe d'insertion de l'élément filaire dans la veine, ce qui ajoute une contrainte supplémentaire pour le praticien.

La mise sous tension réduit également la mobilité autour du lit de bloc opératoire, par peur de toucher l'élément filaire souple.

### Objectif de l'invention

Un objectif de l'invention est de proposer un nouveau dispositif de traitement endoveineux, du type comportant un élément filaire souple pour la délivrance de doses de traitement, lequel dispositif de traitement endoveineux pallie les inconvénients ci-dessus, et notamment permet de réaliser un traitement endoveineux sans que l'élément filaire souple ne soit mis sous tension et permet de réduire les risques de retrait accidentel de l'élément filaire souple en dehors de la veine.

### Résumé de l'invention

Cet objectif est atteint par le dispositif de traitement endoveineux de la revendication 1.

Ce dispositif de traitement endoveineux comporte un système de délivrance d'au moins une dose de traitement, lequel système de délivrance comprend un élément filaire de délivrance de dose de traitement, qui est souple, qui a une partie extrême distale apte à être insérée, au moins sur une partie de sa longueur, longitudinalement dans une veine, et qui permet de délivrer dans une veine au moins une dose de traitement dans la région de l'extrémité de ladite partie extrême distale. Le dispositif de traitement endoveineux comprend en outre un système d'entraînement qui permet d'entraîner l'élément filaire de délivrance de dose de traitement dans au moins une première direction d'entraînement donnée. De manière caractéristique selon l'invention, le dispositif de traitement endoveineux comprend en outre un guide qui est souple sur tout ou partie de sa longueur, qui comporte une partie extrême proximale et une partie extrême distale, et qui permet de guider l'élément filaire de délivrance de dose de traitement, sur une portion de sa longueur, avec une partie extrême proximale, et à l'opposé, une partie extrême distale de l'élément filaire de délivrance de dose de traitement, qui ne sont pas guidées par le guide, et un système de maintien qui permet de maintenir temporairement la partie extrême distale du guide par rapport au corps d'un patient, à proximité de la zone d'insertion de l'élément filaire de délivrance de dose de traitement, l'élément filaire de délivrance de dose de traitement étant apte à coulisser dans le sens de sa longueur par rapport au guide, de manière à permettre l'insertion longitudinalement dans une veine de la partie extrême distale de élément filaire de délivrance de dose de traitement sur au moins une partie de sa longueur lorsque la partie extrême distale du guide est maintenue par rapport au corps d'un patient au moyen du système de maintien, et des moyens de blocage, qui permettent de bloquer axialement la partie extrême proximale du guide, au moins dans la direction d'entrainement de l'élément filaire de délivrance de dose de traitement.

Dans le présent texte, le terme « distal(e) » définit la partie avant d'un élément, et notamment la partie avant du guide ou la partie avant de l'élément filaire de délivrance de dose de traitement, qui est la plus proche du corps humain ou animal lorsqu'on effectue le traitement endoveineux. Le terme « proximal(e) » définit la partie arrière d'un élément, et notamment la partie arrière du guide ou la partie arrière de l'élément filaire de délivrance de dose de traitement, qui est la plus éloignée du corps humain ou animal lorsqu'on effectue le traitement endoveineux.

Plus particulièrement, le dispositif de l'invention peut comporter les caractéristiques additionnelles et optionnelles suivantes, prises isolément, ou en combinaison les unes avec les autres :
- Le système de maintien comporte une pièce de maintien qui est adaptée pour pouvoir être appliquée sur le corps d'un patient, de manière à maintenir temporairement la partie extrême distale (30) du guide par rapport au corps d'un patient, à proximité de la zone d'insertion de l'élément filaire de délivrance de dose de traitement.
- La pièce de maintien comporte une face inférieure permettant d'appliquer la pièce de maintien en contact avec le corps d'un patient, et l'élément filaire de délivrance de dose de traitement est apte à coulisser dans le sens de sa longueur, en étant guidé par la partie extrême distale du guide le long d'un axe de guidage (A) qui ne coupe pas la face inférieure de la pièce de maintien.
- La face inférieure de la pièce de maintien forme une surface d'appui, et dans lequel l'élément filaire de délivrance de dose de traitement est apte à coulisser dans le sens de sa longueur en étant guidé par la partie proximale du guide, le long d'un axe de guidage (A) qui ne coupe pas cette surface d'appui.
- La face inférieure de la pièce de maintien forme une surface d'appui qui est plane ou sensiblement plane, ou est apte à être déformée de manière à former une surface d'appui qui est plane ou sensiblement plane lorsqu'elle est appliquée sur un corps, et l'axe de guidage (A) est sensiblement parallèle à cette surface d'appui de la pièce de maintien.
- L'axe de guidage (A) est incliné par rapport à la surface d'appui de la pièce de maintien d'un angle (α), qui permet l'insertion longitudinalement de l'élément filaire de délivrance de dose de traitement dans une veine, par coulissement de l'élément filaire de délivrance de dose de traitement dans le sens de sa longueur, lorsque la surface d'appui est appliquée contre le corps.
- la pièce de maintien comporte une face inférieure permettant d'appliquer la pièce de maintien en contact avec le corps d'un patient, dans lequel la face inférieure de la pièce de maintien forme une surface d'appui qui est plane ou sensiblement plane, ou est apte à être déformée de manière à former une surface d'appui qui est plane ou sensiblement plane lorsqu'elle est appliquée sur un corps, dans lequel l'élément filaire de délivrance de dose de traitement est apte à coulisser dans le sens de sa longueur, en étant guidé par la partie extrême distale du guide le long d'un axe de guidage (A), qui traverse cette surface d'appui et qui est incliné par rapport à cette surface d'appui d'un angle (α) permettant l'insertion longitudinalement de l'élément filaire de délivrance de dose de traitement dans une veine, par coulissement de l'élément filaire de délivrance de dose de traitement dans le sens de sa longueur, lorsque la surface d'appui est appliquée contre le corps.
- Ledit angle (α) est inférieur ou égal à 60°, préférentiellement inférieur ou égal à 45°, et plus préférentiellement inférieur ou égal à 30°.
- La pièce de maintien est fixée de manière permanente ou peut être fixée de manière permanente ou amovible à la partie extrême distale du guide.
- La pièce de maintien comporte un passage tubulaire traversant dans lequel est enfilée et est fixée la partie extrême distale du guide.
- Le dispositif comporte en outre un cathéter d'introduction qui est apte à être assemblé temporairement avec la partie extrême distale du guide.
- Le guide est conçu de telle sorte qu'une fois enfilé sur l'élément filaire de délivrance de dose de traitement, il subsiste dans le guide une portion de l'élément filaire de délivrance de dose de traitement qui est accessible et peut être manipulée par un opérateur.
- Le système de maintien comporte des moyens de fixation qui permettent de fixer temporairement la partie extrême distale (30) du guide, et le cas échéant la pièce de maintien, sur le corps d'un patient, à proximité de la zone d'insertion de l'élément filaire de délivrance de dose de traitement.
- Les moyens de fixation comportent un adhésif ou une couche adhésive apte à être collé sur la peau.
- Le système d'entraînement est un système de retrait qui permet d'entraîner l'élément filaire de délivrance de dose de traitement vers l'arrière dans une direction d'entraînement (R) permettant le retrait d'une veine de la partie extrême distale de l'élément filaire de délivrance de dose de traitement.
- Les moyens de blocage sont aptes à bloquer axialement la partie extrême proximale du guide également dans la direction (F) opposée à la direction d'entrainement (R) de l'élément filaire de délivrance de dose de traitement.
- L'élément filaire de délivrance de dose de traitement est une fibre optique.
- Le dispositif comprend une source de rayonnement électromagnétique pouvant être couplée à la fibre optique.
- Les moyens de blocage comportent un connecteur en deux parties, une partie fixe qui est fixée sur le système d'entraînement, et une partie amovible qui est fixée à la partie extrême proximale du guide et qui est apte à coopérer avec la partie fixe pour le blocage de la partie extrême proximale du guide.
- Le guide comprend une gaine de guidage souple.
- Le guide comporte plusieurs éléments de guidage assemblés, dont au moins un élément de guidage souple, et plus particulièrement une gaine de guidage souple.

### Brève description des dessins

Les caractéristiques et avantages de l'invention apparaîtront plus clairement à la lecture de la description détaillée ci-après de plusieurs variantes particulières de réalisation de l'invention, lesquelles variantes particulières de réalisation sont décrites à titre d'exemples non limitatifs et non exhaustifs de l'invention, et en référence aux dessins annexés sur lesquels :
- la figure 1 est une vue schématique d'ensemble montrant un exemple de mise en oeuvre d'un dispositif de traitement endoveineux de l'invention, de type laser endoveineux, pour traiter par laser une veine d'une jambe,
- la figure 2 est une vue en coupe transversale d'une première variante de réalisation d'un connecteur pour la partie proximale de la gaine de guidage du dispositif de traitement endoveineux,
- la figure 3 est une vue en coupe transversale d'une deuxième variante de réalisation d'un connecteur pour la partie proximale de la gaine de guidage du dispositif de la figure 1,
- la figure 4 est une vue isométrique d'une première variante de réalisation d'un système de maintien de la partie distale de la gaine de guidage du dispositif de traitement endoveineux,
- La figure 5 est une vue de dessus du système de maintien de la figure 4,
- la figure 6 est une vue isométrique du système de maintien de la figure 4 mis en place et fixé sur une partie de corps humain, avec la fibre optique insérée à travers la peau,
- la figure 7 est une vue en coupe longitudinale de la figure 6, montrant notamment la veine et la fibre optique insérée dans la veine.
- La figure 8 est une vue isométrique illustrant une étape d'insertion de la partie extrême distale de la fibre optique dans une veine, au moyen d'un cathéter d'introduction,
- La figure 9 est une vue isométrique montrant le cathéter d'introduction de la figure 8, retiré de la veine, une fois la partie extrême distale de la fibre optique introduite dans une veine.
- la figure 10 est une vue isométrique d'une deuxième variante de réalisation d'un système de maintien de la partie extrême distale du guide du dispositif de traitement endoveineux,
- la figure 11 est une vue isométrique du système de maintien de la figure 10, lorsque le guide n'est pas assemblé avec la pièce de maintien,
- la figure 12 est une vue isométrique d'une pièce de guidage du guide du dispositif de traitement endoveineux de la figure 10,
- La figure 13 est une vue isométrique de la pièce de maintien du système de maintien de la figure 10,
- la figure 14 est une vue isométrique d'une troisième variante de réalisation d'un système de maintien de la partie extrême distale du guide d'un dispositif de traitement endoveineux,

### Description détaillée

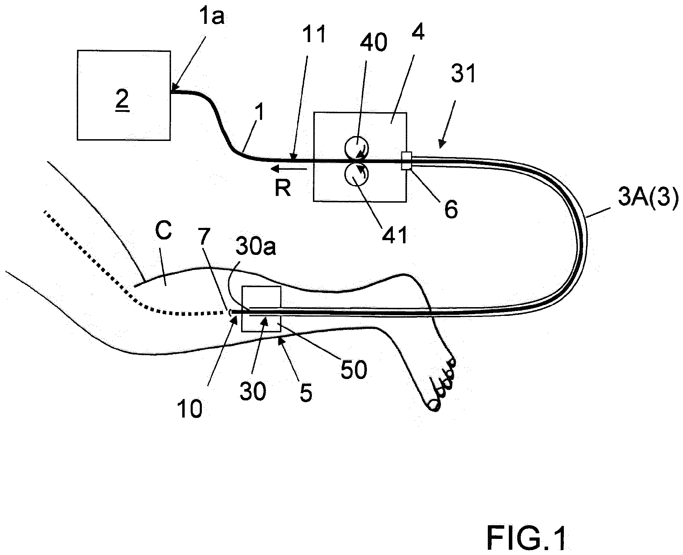

On a représenté de manière schématique sur la figure 1 un dispositif de traitement endoveineux à retrait contrôlé comportant :
- un élément filaire souple 1, qui dans cet exemple particulier est une fibre optique,
- une source de rayonnement électromagnétique 2, de type source laser, qui est couplée à une extrémité proximale 1a de la fibre optique,
- un guide 3, qui dans cet exemple particulier est constitué par une gaine de guidage souple 3A, qui entoure et guide la fibre optique 1 souple sur une portion de sa longueur,
- un système de retrait 4 qui permet de tirer de manière contrôlée et vers l'arrière (direction R) sur la fibre optique 1,

En référence à la figure 1, la gaine de guidage souple 3A comporte une partie extrême proximale 31 et à l'opposé une partie extrême distale 30, qui se termine par une ouverture distale 30a permettant le passage de la fibre optique 1. La fibre optique 1 est enfilée dans la gaine de guidage 3A, de telle sorte que la gaine de guidage 3A entoure et guide la fibre optique 1 sur une portion de sa longueur, avec une partie extrême proximale 11 de la fibre optique 1 et à l'opposé une partie extrême distale 10 de la fibre optique 1 positionnées en dehors de la gaine de guidage 3A. L'extrémité distale de la fibre optique 1 qui permet l'émission du rayonnement électromagnétique dans la veine est ainsi positionnée en dehors de la gaine de guidage 3A, et le praticien peut manipuler manuellement la partie extrême distale 11 de la fibre optique 1.

La longueur de la fibre optique doit être suffisante pour que sa partie extrême proximale 31 puisse être positionnée en dehors du champ opératoire stérile.

La gaine 3A permet un coulissement de la fibre optique 1 avec de préférence un minimum de frottement et est de préférence biocompatible.

Le diamètre intérieur de la gaine 3A doit être également ajusté par rapport au diamètre extérieur de la fibre optique 1, afin de limiter les déplacements radiaux de la fibre optique 1 dans la gaine 3A et permettre une transmission efficace des mouvements longitudinaux. Si l'écart entre le diamètre intérieur de la gaine 3A et le diamètre extérieur de la fibre optique 1, est trop important, on peut occasionner un temps de latence préjudiciable entre le moment où on active le moteur du système de retrait 4 et le moment où on constate le retrait effectif de la fibre par rapport à la gaine. A titre d'exemples non limitatifs et non exhaustifs, avec une fibre optique 1 présentant un diamètre extérieur de 900µm on utilisera une gaine 3A présentant par exemple un diamètre intérieur de 1000µm, et avec une fibre optique 1 présentant un diamètre extérieur de 600µm, on utilisera une gaine 3A présentant par exemple un diamètre intérieur de 700µm.

Différents matériaux peuvent être utilisés pour la gaine 3A, parmi lesquels et de manière non limitative et non exhaustive les matériaux suivants : silicone, polyuréthane, PTFE, PET, ETFE, latex, élastomère thermoplastique.

Le système de retrait 4 est connu en soi et comporte au moins deux galets d'entraînement rotatifs 40, 41, entre lesquels la partie extrême proximale 11 de la fibre optique 1 est positionnée. Le galet 40 est par exemple un galet motorisé et le galet 41 est par exemple un galet monté libre en rotation. Ces deux galets d'entraînement rotatifs 40, 41 permettent d'entraîner par friction la gaine optique 1 vers l'arrière (direction R) à une vitesse contrôlée qui dépend de la vitesse de rotation des galets 40, 41 lors de l'opération de retrait contrôlé de la fibre optique par rapport à la veine à traiter.

Le dispositif de traitement endoveineux comporte en outre :
- un système de maintien 5 qui, dans cette variante particulière de réalisation, permet de fixer la partie extrême distale 30 de la gaine de guidage 3A sur le corps C d'un patient (en l'occurrence sur la figure 1 et de manière non limitative sur une jambe) à proximité du point d'insertion 7 de la fibre optique dans le corps C,
- un système de blocage 6, qui permet de bloquer axialement la partie extrême proximale 31 de la gaine de guidage 3A, par rapport à la fibre optique 1, au moins dans la direction de retrait R de la fibre optique 1, afin de permettre un coulissement relatif vers l'arrière de la fibre optique 1 par rapport à la gaine de guidage 3A.

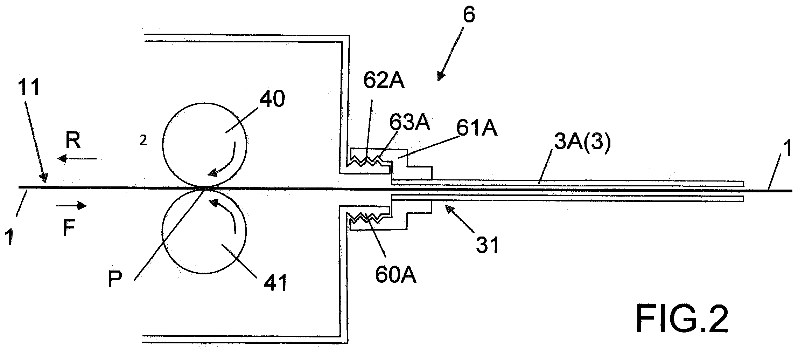

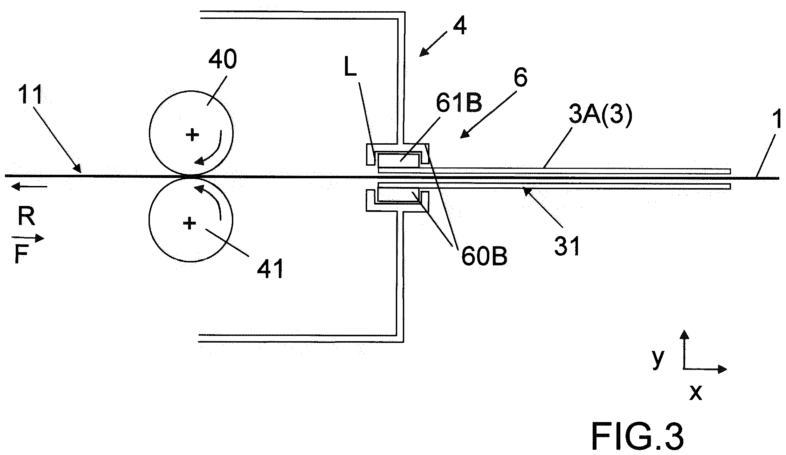

On a représenté sur les figures 2 et 3, deux exemples de réalisation différents d'un système de blocage 6.

Sur la figure 2, le système de blocage 6 est un connecteur en deux parties 60A, 61A. La partie 60A est un embout mâle rigide, qui est prévu sur le système de retrait 4, et qui est fixe par rapport au point de contact P de la fibre optique 1 entre les deux galets d'entraînement 40, 41. Cet embout mâle 60A comporte un filetage 62A. La partie 61A est un connecteur femelle rigide, qui est fixé, par exemple par collage, à la partie extrême proximale 31 de la gaine 3A. Ce connecteur femelle 61A comporte un filetage 63A permettant son vissage sur l'embout maie 60A.

Dans cet exemple de réalisation, lorsque le connecteur femelle 61A est vissé sur l'embout maie 60A, la partie extrême proximale 31 de la gaine est immobilisée dans toutes les directions par rapport au système de retrait 4.

Sur la figure 3, le système de blocage 6 est un connecteur en deux parties 60B, 61B. La partie 60B est un embout femelle rigide, qui est prévu sur le système de retrait 4, et qui est fixe par rapport au point de contact P de la fibre optique 1 entre les deux galets d'entrainement 40, 41. Cet embout femelle 60B forme un logement L. L'autre partie 61B du connecteur est un embout mâle qui est fixé, par exemple par collage, à la partie extrême proximale 31 de la gaine 3A, et qui peut être inséré dans le logement L par translation le long d'un axe perpendiculaire au plan (X,Y) de la figure 3.

Dans cet exemple de réalisation de la figure 3, lorsque l'embout mâle 61B est positionné dans le logement L de l'embout femelle 60B, la partie extrême proximale 31 de la gaine est immobilisée par rapport au système de retrait 4 dans toutes les directions dans le plan (X,Y) perpendiculaire aux axes de rotation A des galets d'entrainement 40, 41.

Plus généralement, le système de blocage 6 devra être conçu de manière à pouvoir bloquer axialement la partie extrême proximale 31 de la gaine de guidage 3A, par rapport à la fibre optique 1, au moins dans la direction de retrait R de la fibre optique 1, et de préférence également dans la direction opposée d'avancement F de la fibre optique 1.

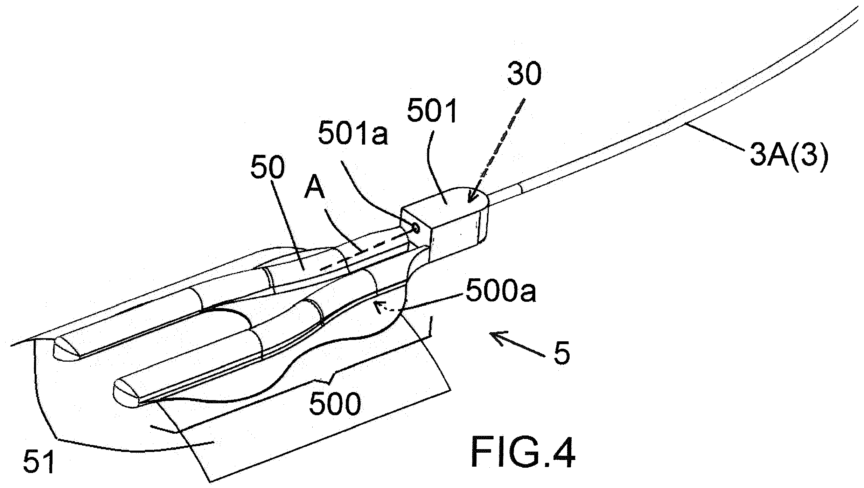

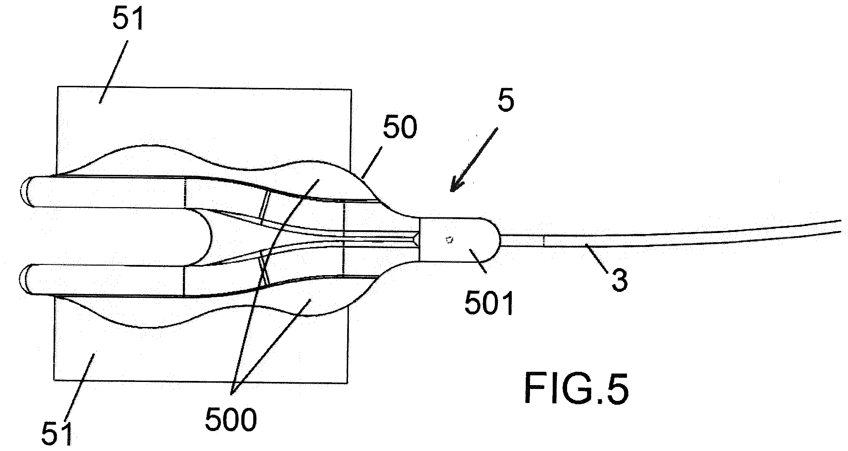

On a représenté sur les figures 4 et 5, un exemple particulier de réalisation d'un système de maintien 5 conforme à une première variante particulière de réalisation de l'invention.

Ce système de maintien 5 comporte une pièce de maintien 50, qui est par exemple une pièce moulée en plastique. Cette pièce de maintien 50 comporte une partie avant principale 500 en forme de spatule dont la face inférieure 500a forme une surface d'appui sensiblement plane ou légèrement courbe et est destinée à être appliquée contre le corps humain. Cette partie avant principale 500 peut être rigide ou légèrement flexible de manière à pouvoir adapter la courbure de sa face inférieure 500a à la partie de corps humain C.

Cette pièce de maintien 50 comporte également en partie arrière un connecteur 501, qui permet de fixer la partie extrême distale 30 de la gaine 3A à la pièce de maintien 50. Dans la variante particulière illustrée, ce connecteur 501 comporte un passage tubulaire traversant 501a d'axe central longitudinal A dans lequel est enfilée et est fixée, par exemple par collage, la partie extrême distale 30 de la gaine 3A.

L'axe central longitudinal A de la partie distale 30 de la gaine de guidage 3A qui se confond avec l'axe central longitudinal du passage traversant 501a forme un axe de guidage de la fibre optique 1 qui ne coupe pas la surface d'appui formée par la face inférieure 500a de la pièce de maintien 50. La fibre optique 1 est ainsi apte à coulisser dans le sens de sa longueur par rapport à la pièce de maintien 50 et à la gaine de guidage 3A, en étant guidée par la partie distale 30 de la gaine de guidage 3A le long de cet axe de guidage A.

Cette orientation de l'axe de guidage A de la partie distale 30 de la gaine de guidage 3A, par rapport à la surface d'appui formée par la face inférieure 500a de la pièce de maintien 50, permet ainsi l'insertion longitudinalement dans une veine V de la partie proximale 10 de la fibre optique 1, sur au moins une partie de sa longueur, lorsque la partie extrême proximale 30 de la gaine de guidage 3A est maintenue par rapport au corps d'un patient au moyen de la pièce de maintien 50.

Plus particulièrement, mais de manière non limitative, dans cet exemple l'axe de guidage A est sensiblement parallèle à la surface d'appui formée par la face inférieure 500a de la pièce de maintien 50.

Le système de maintien 5 comporte également un moyen de fixation 51, qui permet de fixer temporairement au corps humain C la pièce de maintien 50, et de ce fait la partie extrême distale 30 de la gaine de guidage 3, pendant la durée du traitement endoveineux. Dans la variante particulière illustrée, ce moyen de fixation est un adhésif double-face 51, qui est collé contre la face inférieure 500a de la pièce de maintien 50 et qui est apte à être collé de manière amovible sur la peau.

Cet adhésif double face 51 peut être remplacé par tout moyen équivalent, permettant de fixer temporairement la partie extrême distale 30 de la gaine de guidage 3, au corps humain C pendant la durée du traitement endoveineux. Cet adhésif double-face 51 peut par exemple être remplacé par un élastique ou une sangle Velcro® ou autre, qui est apte à entourer la partie de corps humain où est localisée la partie de veine à traiter et qui permet de maintenir temporairement la pièce de maintien 50 appliquée contre cette partie de corps humain.

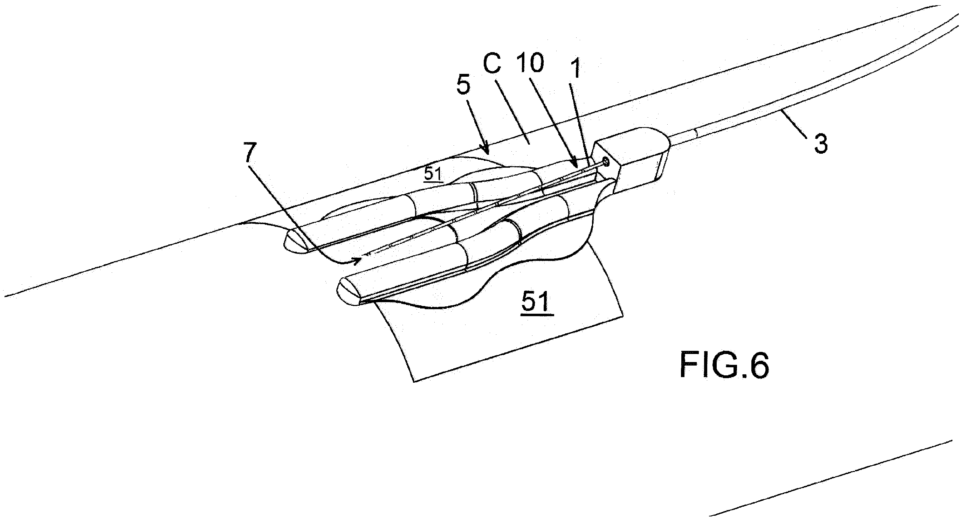

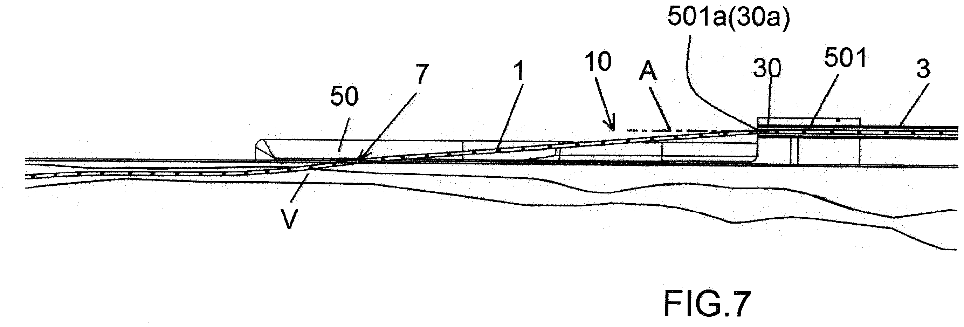

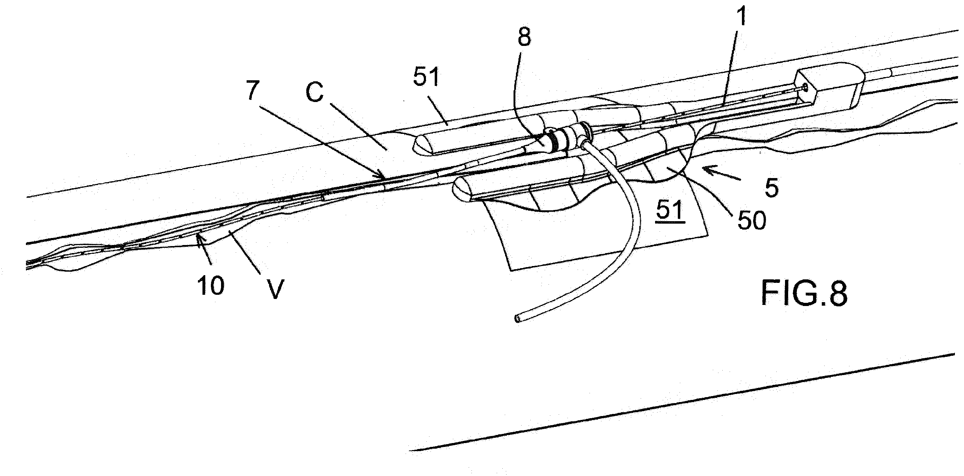

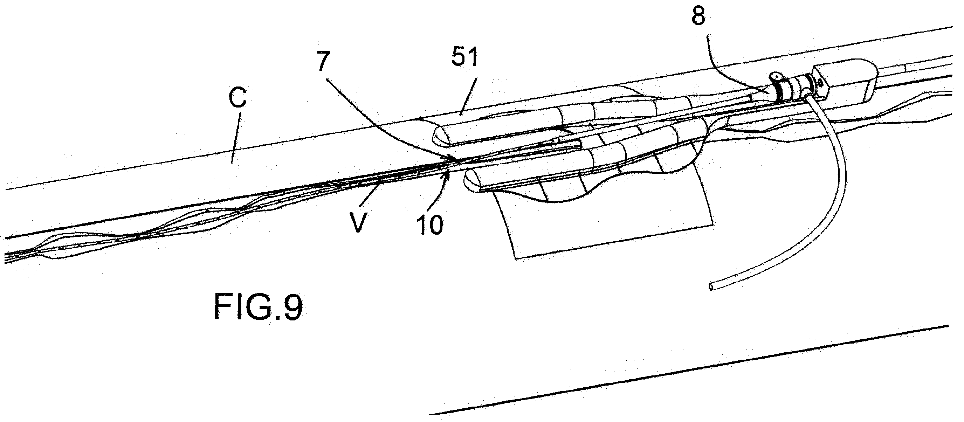

Un exemple particulier de mise en oeuvre du dispositif de traitement endoveineux va à présent être détaillé, en référence aux figures 4 à 7.
(a) On enfile et on fait coulisser manuellement la fibre optique 1 dans la gaine de guidage 3A, jusqu'à ce que la partie extrême distale 10 de la fibre optique 1 sorte par l'ouverture extrême distale 30a de la gaine de guidage 3A et soit positionnée en dehors de la gaine de guidage 3A et du passage traversant 501a de la pièce de maintien 50.
(b) On positionne la partie extrême proximale 11 de la fibre optique 1 dans le système de retrait 4 et on bloque la partie extrême proximale 31 de la gaine 3 par rapport au système de retrait 4 à l'aide du système de blocage 6 précédemment décrit. Puis on couple l'extrémité proximale 1a de la fibre optique 1 à la sortie de la source de rayonnement électromagnétique 2
(c) On fixe la partie extrême distale 30 de la gaine par rapport au corps C, en fixant la pièce de maintien 50 sur le corps humain C à proximité du point d'insertion 7 de la fibre optique 1.
(d) On enfonce de manière usuelle, à travers la peau et dans la veine V à traiter, la partie e d'une aiguille creuse, communément appelée aiguille de ponction, dont la pointe distale est localisée par ultrasons au moyen d'une sonde échographique. Le point d'insertion de cette aiguille correspond au point d'insertion 7 susvisé.
(e) On insère un fil-guide dans cette aiguille creuse jusque dans la veine à traiter, puis on retire l'aiguille.
(f) On enfile un cathéter d'introduction 8 sur le fil-guide jusqu'à l'entrée de la veine V et on retire le fil-guide.
(g) Une fois le cathéter d'introduction 8 mis en place (figure 8), on insère la partie extrême distale 10 de la fibre optique 1, qui dépasse en dehors de la partie extrême distale 30 de la gaine 3A, dans le cathéter d'introduction 8 et on fait coulisser la fibre optique 1 vers l'avant par rapport à la gaine 3A, jusqu'à ce que l'extrémité de la partie extrême distale 10 de fibre optique 1 pénètre longitudinalement dans la veine V et progresse dans la veine V jusqu'à la zone à traiter la plus éloignée du point d'insertion 7. Pendant cette opération, le moteur d'entraînement du galet 40 est débrayé.
(h) Une fois la fibre optique 1 introduite et positionnée dans la veine V, on retire le cathéter 8 de la veine V en le faisant coulisser vers l'arrière sur la fibre optique 1 (figure 9). Eventuellement on retire le cathéter 8 de la fibre optique 1, par exemple en le fendant en deux dans le cas d'un cathéter déchirable. Dans une autre variante, le cathéter peut être retiré à l'issue de la procédure de traitement.

Le praticien peut alors pratiquer de manière usuelle le traitement endoveineux en actionnant manuellement la source laser 2, afin d'émettre dans la veine un rayonnement électromagnétique dans la région de l'extrémité de la partie distale de la fibre optique 1 et en commandant le retrait continu ou pas à pas de la fibre optique 1 au moyen du système de retrait 4.

Grâce à la gaine de guidage 3A, dont la partie extrême distale 30 est fixée temporairement au corps C, à proximité du point d'insertion 7 de la fibre optique 1, et dont la partie extrême proximale 31 est bloquée axialement par rapport à la fibre optique 1 au moyen du system de blocage 6, le traitement endoveineux peut avantageusement être réalisé sans que la fibre optique 1 ne soit tendue et en réduisant les risques de déplacements accidentels de la fibre optique par rapport à la veine en cours de traitement.

Une fois le traitement laser terminé, la fibre optique 1 est totalement retirée de la veine et le système de maintien 5 est désolidarisé du corps humain.

Le système de retrait 4 peut d'une manière plus générale être remplacé par tout système d'entraînement permettant d'entraîner l'élément filaire 1 de délivrance de doses de traitement dans au moins une direction d'entraînement R donnée. Ce système d'entraînement 4 du dispositif n'est pas nécessairement motorisé, mais pourrait être un système d'entraînement actionné manuellement.

Dans le cadre de l'invention, la gaine de guidage 3A peut être remplacée par tout guide souple équivalent remplissant la même fonction de guidage que la gaine 3A. Par exemple, et de manière non exhaustive, la gaine 3A peut être remplacée par un guide souple en forme de gouttière, et ayant par exemple une section transversale en U, ou par un guide filaire souple torsadé autour de la fibre optique 1 ou équivalent, ou par un guide souple qui est aimanté pour permettre sa solidarisation avec l'élément filaire 1 de délivrance des doses de traitement.

Le système de maintien 5 peut comporter uniquement la pièce de maintien 50 ou équivalent et peut ne pas comporter le moyen de fixation 51 ou équivalent. Dans ce cas, la pièce de maintien 50 est utilisée pour maintenir temporairement de manière manuelle la partie extrême distale 30 du guide 3 par rapport au corps du patient à proximité du point d'insertion 7 de l'élément filaire 1 de délivrance de dose de traitement.

Le système de maintien peut comporter des moyens de fixation permettant de fixer temporairement la partie extrême distale 30 du guide 3 sur le corps d'un patient, à proximité de la zone d'insertion 7 de l'élément filaire 1 de délivrance de dose de traitement, sans mise en oeuvre de la pièce de maintien 50. Par exemple, le système de maintien peut être formé de un ou plusieurs adhésifs aptes à être appliqués directement sur la partie extrême distale 30 du guide 3 et à être collés sur le corps du patient pour fixer temporairement la partie extrême distale 30 du guide 3 par rapport au corps du patient à proximité du point d'insertion 7 de l'élément filaire 1 de délivrance de dose de traitement.

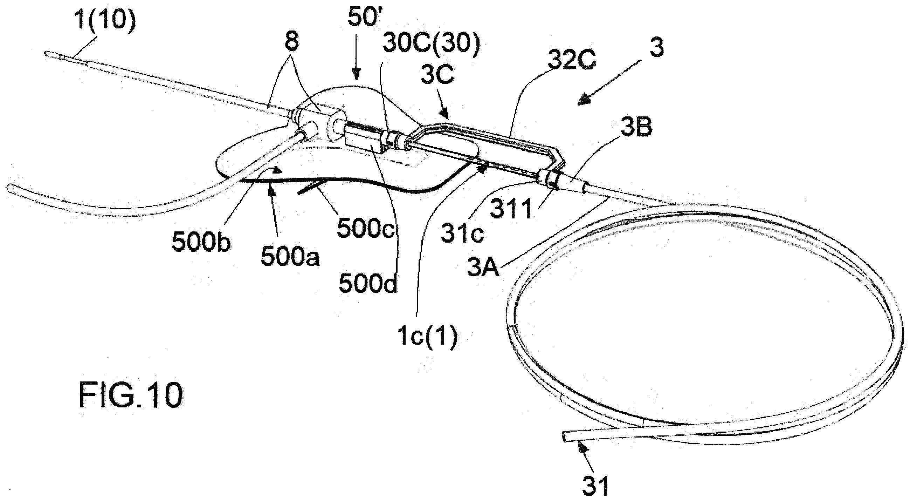

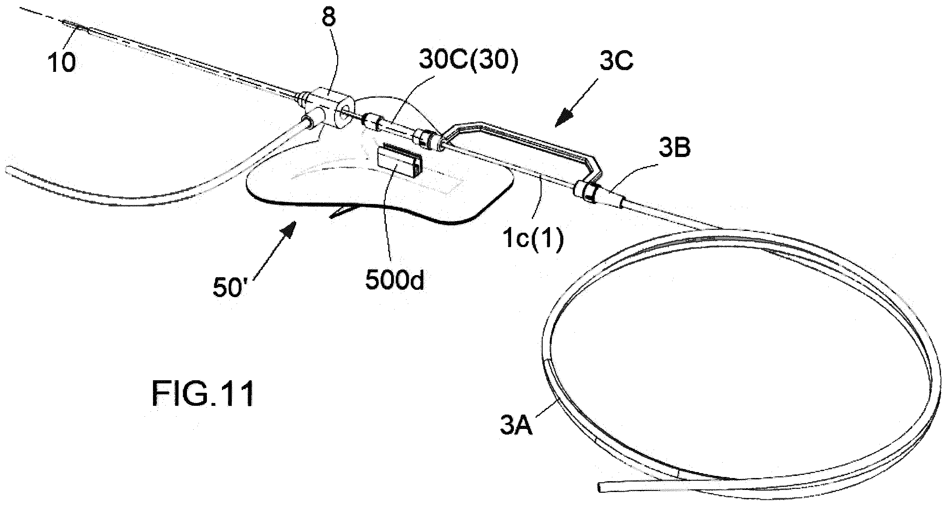

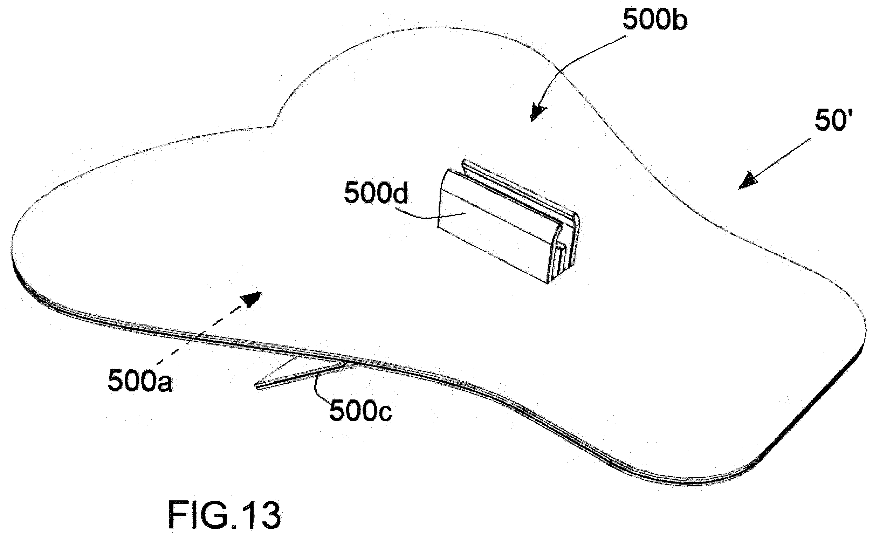

On a représenté sur les figures 10 à 13 une deuxième variante de réalisation de l'invention dans laquelle le guide 3 comporte plusieurs éléments de guidage assemblés, et le système de maintien 5 comporte une pièce de maintien 50' (figure 13) qui est conçue de manière à pouvoir être assemblée de manière amovible avec le guide 3 (figures 10 et 11).

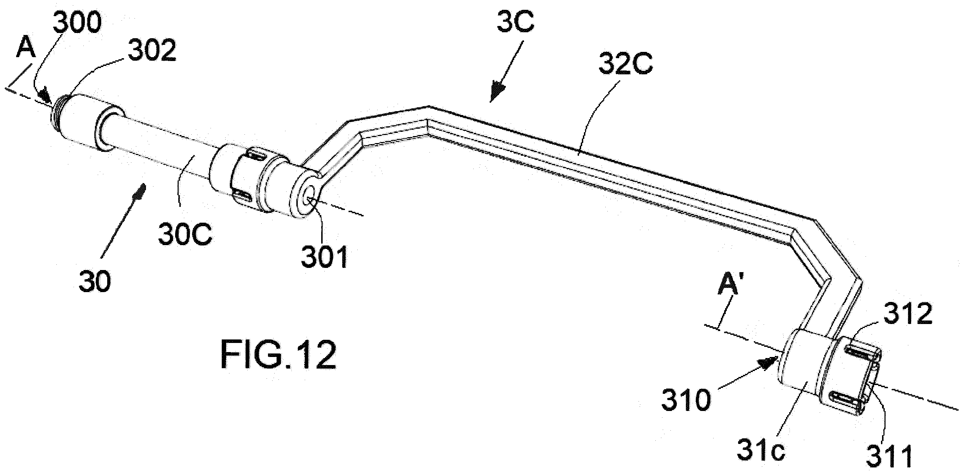

Ce guide 3 comporte une gaine de guidage souple 3A équipée à une extrémité d'un connecteur 3B et une pièce de guidage 3C rigide, qui est représentée seule sur la figure 12, et qui est fixée rigidement (figure 10) à l'extrémité proximale de la gaine souple 3A au moyen du connecteur 3B.

Dans cette variante, comme pour la première variante précédemment décrite, la partie extrême proximale 31 de la gaine de guidage 3A peut être bloquée axialement par rapport à la fibre optique 1 au moyen d'un système de blocage 6, précédemment décrit et non représenté sur les figures 10 à 13.

La pièce de guidage 3C est par exemple une pièce en plastique.

Cette pièce de guidage 3C comporte :
- un élément tubulaire de guidage avant 30C, qui forme ladite partie extrême distale 30 du guide 3, et qui définit un axe de guidage A (figure 12) de la fibre optique 1, une ouverture avant 300 et une ouverture arrière 301 pour le passage de la fibre optique 1,
- un élément tubulaire de guidage arrière 31C, qui définit un axe de guidage A' (figure 12) aligné avec l'axe de guidage A, une ouverture avant 310 et une ouverture arrière 311 pour le passage de la fibre optique 1,
- une élément de liaison 32C rigide, en forme de U, reliant l'élément tubulaire de guidage avant 30C à l'élément tubulaire de guidage arrière 31C de manière déportée par rapport aux axes de guidage alignés A et A'.

L'élément tubulaire de guidage arrière 31C comporte en outre un connecteur 312 apte à coopérer, et notamment à être emboîté avec serrage, avec le connecteur 3B fixé à l'extrémité de la gaine de guidage 3A.

L'élément tubulaire de guidage avant 30C comporte un moyen d'assemblage 302 (figure 12), en l'espèce sous la forme d'un pas de vis, pour son assemblage temporaire, notamment par vissage, avec un cathéter d'introduction 8 (figure 10).

En référence aux figures 10 et 11, la pièce de guidage 3C est assemblée à l'extrémité distale de la gaine de guidage 3A au moyen des connecteurs 3B et 311, en étant enfilée sur la fibre optique 1. La fibre optique 1 est apte à coulisser dans le sens de sa longueur par rapport à la gaine de guidage 3A et par rapport la pièce de guidage 3C, en étant guidée en dehors de la gaine de guidage 3A par les deux éléments tubulaires de guidage 30C et 31C.

En référence à la figure 13, la pièce de maintien 50' est une pièce de faible épaisseur, par exemple une pièce moulée en plastique dont la face inférieure 500a forme une surface d'appui sensiblement plane ou légèrement courbe et est destinée à être appliquée contre le corps humain. Cette pièce de maintien 50 peut être rigide ou légèrement flexible de manière à pouvoir adapter la courbure de sa face inférieure 500a à la partie de corps humain C.

La face inférieure 500a de cette pièce de maintien 50' peut être revêtue d'une couche adhésive couvrant tout ou partie de sa surface et temporairement protégée par un film de protection 500c pouvant être retiré à la main avant usage. Cette couche adhésive forme un moyen de fixation permettant de fixer temporairement la pièce de maintien 50' appliquée sur le corps d'un patient à proximité du point d'insertion 7 de la fibre optique 1.

Cette pièce de maintien 50 comporte également sur sa face supérieure 500b, un moyen d'assemblage 500d, par exemple sous la forme d'un clip élastiquement déformable, qui permet un assemblage rapide de la pièce de maintien 50' avec l'élément tubulaire de guidage avant 30C de la pièce de guidage 3C du guide 3, c'est-à-dire avec la partie extrême distale 30 du guide 3.

En référence à la figure 10, la pièce de maintien 50' est apte à être assemblée avec la partie extrême distale 30 (élément tubulaire de guidage avant 30C) du guide 3, de telle sorte que l'axe de guidage A (figure 12) de la partie extrême distale 30 du guide 3, ne coupe pas la surface d'appui formée par la face inférieure 500a de la pièce de maintien 50'. La fibre optique 1 est ainsi apte à coulisser dans le sens de sa longueur par rapport à la pièce de maintien 50' et au guide 3, en étant guidée par la partie distale 30 du guide 3 le long de cet axe de guidage A qui ne coupe pas la surface d'appui formée par la face inférieure 500a de la pièce de maintien 50'.

Cette orientation de l'axe de guidage A de la partie distale 30 (élément tubulaire de guidage avant 30C) du guide 3, par rapport à la surface d'appui formée par la face inférieure 500a de la pièce de maintien 50', permet ainsi l'insertion longitudinalement dans une veine V de la partie distale 10 de la fibre optique 1, sur au moins une partie de sa longueur, lorsque la partie extrême distale 30 du guide 3 est maintenue par rapport au corps d'un patient au moyen de la pièce de maintien 50'.

Plus particulièrement, mais de manière non limitative, dans cet exemple l'axe de guidage A ne coupe pas la surface d'appui formée par la face inférieure 500a, et plus particulièrement est sensiblement parallèle à cette surface d'appui qui est plane ou sensiblement plane.

Dans la variante de réalisation des figures 10 à 13, la fibre optique 1 est avantageusement accessible pour un opérateur dans sa portion 1c (figure 10) située entre l'élément tubulaire de guidage avant 30C et l'élément tubulaire de guidage arrière 31C, ce qui permet avantageusement à un opérateur de manipuler la fibre optique 1 à la main, pour le cas échéant procéder à des ajustements de sa position dans une veine V, en tirant localement sur la fibre optique 1 vers l'arrière ou en poussant localement la fibre optique 1 vers avant en direction de la veine.

Un exemple particulier de mise en oeuvre du dispositif de traitement endoveineux de la figure 10 va être à présent détaillé, le cathéter d'introduction 8 n'étant pas fixé à la partie extrême proximale 30 du guide 3, et la partie extrême distale 10 de la fibre optique 1 dépassant de la partie extrême distale 30 du guide 3.
(a) on positionne la partie extrême proximale 11 de la fibre optique 1 dans le système de retrait 4 et on bloque la partie extrême proximale 31 de la gaine 3 par rapport au système de retrait 4 à l'aide du système de blocage 6 précédemment décrit. Puis on couple l'extrémité proximale 1a de la fibre optique 1 à la sortie de la source de rayonnement électromagnétique 2.
(b) On fixe la partie extrême distale 30 du guide 3 par rapport au corps C, en fixant la pièce de maintien 50' sur le corps humain C à proximité du point d'insertion 7 de la fibre optique 1.
(c) On enfonce de manière usuelle, à travers la peau et dans la veine V à traiter, la partie distale d'une aiguille creuse, communément appelée aiguille de ponction, dont la pointe est localisée par ultrasons au moyen d'une sonde échographique. Le point d'insertion de cette aiguille correspond au point d'insertion 7 susvisé.
(d) On insère un fil-guide dans cette aiguille creuse jusque dans la veine à traiter, puis on retire l'aiguille.
(e) On enfile le cathéter d'introduction 8 sur le fil-guide jusqu'à l'entrée de la veine V et on retire le fil-guide.
(f) Une fois le cathéter d'introduction 8 mis en place, on insère la partie extrême distale 10 de la fibre optique 1, qui dépasse en dehors de la partie extrême distale 30 du guide 3, dans le cathéter d'introduction 8 et on fait coulisser la fibre optique 1 vers l'avant par rapport à la gaine 3A, jusqu'à ce que l'extrémité de la partie extrême distale 10 de fibre optique 1 pénètre longitudinalement dans la veine V et progresse dans la veine V jusqu'à la zone à traiter la plus éloignée du point d'insertion 7. Pendant cette opération, le moteur d'entraînement du galet 40 est débrayé.
(g) Une fois la fibre optique 1 introduite et positionnée dans la veine V, on fait coulisser le cathéter 8 vers l'arrière sur la fibre optique 1 et on le fixe à la partie extrême distale 30 du guide 3 tel qu'illustré sur la figure 10. Le cathéter 8 sera retiré à l'issue de la procédure de traitement.

L'étape (g) est facultative. L'étape (b) peut être effectuée après l'étape (f).

Le praticien peut alors pratiquer de manière usuelle le traitement endoveineux en actionnant manuellement la source laser 2, afin d'émettre dans la veine un rayonnement électromagnétique dans la région de l'extrémité de la partie distale de la fibre optique 1 et en commandant le retrait continu ou pas à pas de la fibre optique 1 au moyen du système de retrait 4.

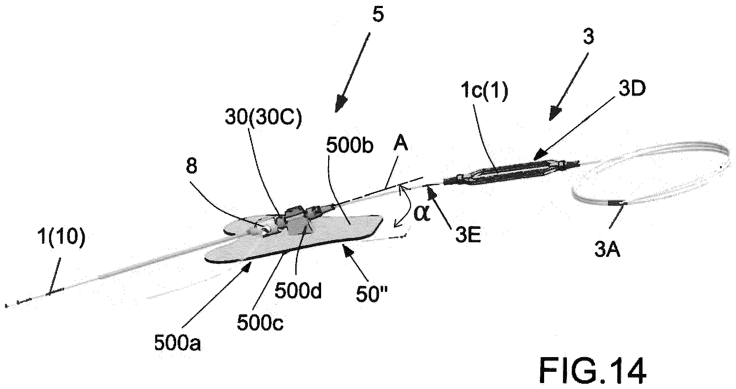

On a représenté sur la figure 14, une troisième variante de réalisation de l'invention.

Dans cette variante la surface d'appui qui est formée par la face inférieure 500a de la pièce de maintien 50" est plane ou sensiblement plane, et la partie extrême distale 30 du guide 3 constituée par l'élément tubulaire de guidage avant 30C est assemblée avec la pièce de maintien 50" au moyen du clip d'assemblage 500d, de telle sorte que, comme pour la deuxième variante susvisée, l'axe de guidage A de cette partie extrême distale 30 du guide 3 ne coupe pas cette surface d'appui.

A la différence de la deuxième variante susvisée, cet axe de guidage A n'est pas sensiblement parallèle la surface d'appui qui est formée par la face inférieure 500a de la pièce de maintien 50', mais est incliné par rapport à cette surface d'appui 500a d'un angle α qui permet l'insertion longitudinalement de la fibre optique dans une veine, par coulissement vers l'avant de la fibre optique 1 dans le sens de sa longueur, lorsque la surface d'appui 500a est appliquée contre le corps d'un patient.

De préférence cet angle α est inférieur ou égal à 60° et plus préférentiellement est inférieur ou égal à 45°, et plus préférentiellement encore est inférieur ou égal à 30°. Cette inclinaison de l'axe de guidage A permet de faciliter l'introduction de la fibre optique 1 dans une veine comparativement à la deuxième variante précédemment décrite.

Dans une autre variante de réalisation (non représentée), l'axe de guidage A peut traverser la surface d'appui qui est formée par la face inférieure 500a de la pièce de maintien 50". Dans ce cas, la face inférieure 500a de la pièce de maintien 50" définissant une surface d'appui qui est plane ou sensiblement plane, l'axe de guidage est incliné par rapport à cette surface d'appui 500a d'un angle α qui permet l'insertion longitudinalement de la fibre optique dans une veine, par coulissement vers l'avant de la fibre optique dans le sens de sa longueur, lorsque la surface d'appui 500a est appliquée contre le corps d'un patient. De préférence cet angle α est inférieur ou égal à 60°, préférentiellement inférieur ou égal à 45° plus préférentiellement inférieur ou égal à 30°.

Il convient de noter que dans le cadre de l'invention, la face inférieure 500a de la pièce de maintien 50, 50' ou 50" peut être rigide et définir une surface d'appui sur le corps qui est plane ou sensiblement plane ou peut être déformable lorsqu'elle est appuyée sur le corps et définir à l'état déformé, lorsqu'elle est appuyée sur le corps, une surface d'appui sur le corps qui est plane ou sensiblement plane.

Dans cette troisième variante de réalisation, la gaine de guidage souple 3A est reliée à l'élément tubulaire de guidage avant 30C (formant la partie extrême proximale du guide 3) par une pièce de guidage 3D rigide, par exemple en plastique, enfilée sur la fibre optique 1 et fixée à l'extrémité proximale de la gaine 3A, et par une gaine flexible 3E de faible longueur, enfilée sur la fibre optique 1 et fixée à l'extrémité proximale de la pièce de pièce de liaison 3D et à l'extrémité proximale de l'élément tubulaire de guidage avant 30C. La flexibilité de cette gaine 3E permet de limiter les efforts transmis à la partie extrême distale 30 du guide 3 lors des manipulations du dispositif endoveineux.

La pièce de liaison 3D est conçue de telle sorte qu'une portion 1c de la fibre optique 1 est à nue et est ainsi avantageusement accessible pour un opérateur (figure 14), ce qui permet avantageusement à un opérateur de manipuler la fibre optique 1 à la main pour le cas échéant de procéder à des ajustements de sa position dans une veine V, en tirant localement sur cette portion 1c la fibre optique vers l'arrière ou en poussant localement cette portion 1c la fibre optique 1 vers avant en direction de la veine.

Dans la variante les figures annexées, la face inférieure 500a de pièce de maintien 50, 50', 50" forme une surface d'appui continue. Dans une autre variante, cette surface d'appui pourrait est discontinue et définie par des éléments d'appuis espacés.

L'invention n'est pas limitée à un dispositif de traitement endoveineux par laser. Dans d'autres variantes de réalisation couvertes par l'invention, la fibre optique peut être remplacée par un élément filaire (plein ou creux) par exemple de type câble ou sonde souple ou canule souple. Le traitement n'est pas nécessairement un traitement par laser, mais peut être tout traitement par délivrance de doses de traitement dans la veine, et notamment de doses d'énergie, délivrées par exemple sous la forme d'un rayonnement électromagnétique, au moyen d'ondes sonores ou ultrasonores, d'ondes radiofréquences, ou de doses d'énergie thermique délivrées par rayonnement et/ou par contact, ou de doses d'un produit, par exemple liquide, semi-liquide ou mousseux, permettant un traitement de la veine.

## Revendications

1. Dispositif de traitement endoveineux comportant un système de délivrance (1 ; 2) d'au moins une dose de traitement, lequel système de délivrance (1 ; 2) comprend un élément filaire (1) de délivrance de dose de traitement, qui est souple, qui a une partie extrême distale (10) apte à être insérée, au moins sur une partie de sa longueur, longitudinalement dans une veine (V), et qui permet de délivrer dans une veine (V) au moins une dose de traitement dans la région de l'extrémité de ladite partie extrême distale (10), le dispositif de traitement endoveineux comprenant en outre un système d'entraînement (4) qui permet d'entraîner l'élément filaire (1) de délivrance de dose de traitement dans au moins dans une première direction d'entraînement (R) donnée, **caractérisé en ce qu'**il comprend en outre un guide (3) qui est souple sur tout ou partie de sa longueur, qui comporte une partie extrême proximale (31) et une partie extrême distale (30), et qui permet de guider l'élément filaire (1) de délivrance de dose de traitement, sur une portion de sa longueur, avec une partie extrême proximale (11), et à l'opposé, une partie extrême distale (10) de l'élément filaire (1) de délivrance de dose de traitement, qui ne sont pas guidées par le guide (3), et **en ce qu'**il comprend en outre un système de maintien (5) qui permet de maintenir temporairement la partie extrême distale (30) du guide (3) par rapport au corps d'un patient, à proximité de la zone d'insertion (7) de l'élément filaire (1) de délivrance de dose de traitement, l'élément filaire (1) de délivrance de dose de traitement étant apte à coulisser dans le sens de sa longueur par rapport au guide (3), de manière à permettre l'insertion longitudinalement dans une veine (V) de la partie extrême distale (10) de élément filaire (1) de délivrance de dose de traitement sur au moins une partie de sa longueur lorsque la partie extrême distale (30) du guide (3) est maintenue par rapport au corps d'un patient au moyen du système de maintien (5), et des moyens de blocage (6), qui permettent de bloquer axialement la partie extrême proximale (31) du guide (3), au moins dans la direction d'entrainement (R) de l'élément filaire (1) de délivrance de dose de traitement.

2. Dispositif selon la revendication 1, dans lequel le système de maintien (5) comporte une pièce de maintien (50 ; 50' ; 50"), qui est adaptée pour pouvoir être appliquée sur le corps d'un patient, de manière à maintenir temporairement la partie extrême distale (30) du guide (3) par rapport au corps d'un patient, à proximité de la zone d'insertion (7) de l'élément filaire (1) de délivrance de dose de traitement.

3. Dispositif selon la revendication 2, dans lequel la pièce de maintien (50 ; 50' ; 50") comporte une face inférieure (500a) permettant d'appliquer la pièce de maintien (50 ; 50' ; 50") en contact avec le corps d'un patient, et dans lequel l'élément filaire (1) de délivrance de dose de traitement est apte à coulisser dans le sens de sa longueur, en étant guidé par la partie extrême distale (30) du guide (3) le long d'un axe de guidage (A) qui ne coupe pas la face inférieure (500a) de la pièce de maintien (50 ; 50').

4. Dispositif selon la revendication 3, dans lequel la face inférieure (500a) de la pièce de maintien (50 ; 50' ; 50") forme une surface d'appui, et dans lequel l'élément filaire (1) de délivrance de dose de traitement est apte à coulisser dans le sens de sa longueur en étant guidé par la partie proximale du guide (3), le long d'un axe de guidage (A) qui ne coupe pas cette surface d'appui.

5. Dispositif selon la revendication 4, dans lequel la face inférieure (500a) de la pièce de maintien (50 ; 50' ; 50") forme une surface d'appui qui est plane ou sensiblement plane, ou est apte à être déformée de manière à former une surface d'appui qui est plane ou sensiblement plane lorsqu'elle est appliquée sur un corps, et l'axe de guidage (A) est sensiblement parallèle à cette surface d'appui de la pièce de maintien (50 ; 50') ou dans lequel l'axe de guidage (A) est incliné par rapport à la surface d'appui de la pièce de maintien (50") d'un angle (a), qui permet l'insertion longitudinalement de l'élément filaire (1) de délivrance de dose de traitement dans une veine, par coulissement de l'élément filaire (1) de délivrance de dose de traitement dans le sens de sa longueur, lorsque la surface d'appui est appliquée contre le corps.

6. Dispositif selon la revendication 2, dans lequel la pièce de maintien comporte une face inférieure (500a) permettant d'appliquer la pièce de maintien en contact avec le corps d'un patient, dans lequel la face inférieure (500a) de la pièce de maintien forme une surface d'appui qui est plane ou sensiblement plane, ou est apte à être déformée de manière à former une surface d'appui qui est plane ou sensiblement plane lorsqu'elle est appliquée sur un corps, dans lequel l'élément filaire (1) de délivrance de dose de traitement est apte à coulisser dans le sens de sa longueur, en étant guidé par la partie extrême distale (30) du guide (3) le long d'un axe de guidage (A), qui traverse cette surface d'appui et qui est incliné par rapport à cette surface d'appui d'un angle (a) permettant l'insertion longitudinalement de l'élément filaire (1) de délivrance de dose de traitement dans une veine, par coulissement de l'élément filaire (1) de délivrance de dose de traitement dans le sens de sa longueur, lorsque la surface d'appui est appliquée contre le corps.

7. Dispositif selon l'une quelconque des revendications 5 ou 6, dans lequel ledit angle (a) est inférieur ou égal à 60°, préférentiellement inférieur ou égal à 45°, et plus préférentiellement inférieur ou égal à 30°.

8. Dispositif selon l'une quelconque des revendications 2 à 7, dans lequel la pièce de maintien (50 ; 50' ; 50") est fixée de manière permanente ou peut être fixée de manière permanente ou amovible à la partie extrême distale (30) du guide (3).

9. Dispositif selon l'une quelconque des revendications précédentes, comportant en outre un cathéter d'introduction (8) qui est apte à être assemblé temporairement avec la partie extrême distale (30) du guide (3).

10. Dispositif selon l'une quelconque des revendications précédentes, dans lequel le guide (3) est conçu de telle sorte qu'une fois enfilé sur l'élément filaire (1) de délivrance de dose de traitement, il subsiste dans le guide (3) une portion (1 c) de l'élément filaire (1) de délivrance de dose de traitement qui est accessible et peut être manipulée par un opérateur.

11. Dispositif selon l'une quelconque des revendications précédentes, dans lequel le système de maintien (5) comporte des moyens de fixation (51) qui permettent de fixer temporairement la partie extrême distale (30) du guide (3), et le cas échéant la pièce de maintien (50 ; 50' ; 50"), sur le corps d'un patient, à proximité de la zone d'insertion (7) de l'élément filaire (1) de délivrance de dose de traitement et de préférence dans lequel les moyens de fixation (51) comportent un adhésif ou une couche adhésive apte à être collé sur la peau.

12. Dispositif selon l'une quelconque des revendications précédentes, dans lequel le système d'entraînement (4) est un système de retrait qui permet d'entraîner l'élément filaire (1) de délivrance de dose de traitement vers l'arrière dans une direction d'entraînement (R) permettant le retrait d'une veine de la partie extrême distale (10) de l'élément filaire (1) de délivrance de dose de traitement.

13. Dispositif selon l'une quelconque des revendications précédentes, dans lequel les moyens de blocage (6) sont aptes à bloquer axialement la partie extrême proximale (31) du guide (3) également dans la direction (F) opposée à la direction d'entrainement (R) de l'élément filaire (1) de délivrance de dose de traitement.

14. Dispositif selon l'une quelconque des revendications précédentes, dans lequel l'élément filaire (1) de délivrance de dose de traitement est une fibre optique, et de préférence comprenant une source de rayonnement électromagnétique (2) pouvant être couplée à la fibre optique (1).

15. Dispositif selon l'une quelconque des revendications précédentes, dans lequel les moyens de blocage (6) comportent un connecteur en deux parties, une partie fixe (60A; 61A) qui est fixée sur le système d'entraînement (4), et une partie amovible (60B ; 61B) qui est fixée à la partie extrême proximale (31) du guide (3) et qui est apte à coopérer avec la partie fixe (60A ; 61A) pour le blocage de la partie extrême proximale (31) du guide (3).

16. Dispositif selon l'une quelconque des revendications précédentes, dans lequel le guide (3) comprend une gaine de guidage souple (3A), ou comprend plusieurs éléments de guidage assemblés, dont au moins un élément de guidage souple (3A), et plus particulièrement une gaine de guidage souple (3A).

## Patentansprüche

1. Vorrichtung zur endovenösen Behandlung mit einem Abgabesystem (1; 2) zur Abgabe mindestens einer Behandlungsdosis, wobei das Abgabesystem (1; 2) ein faserförmiges Element (1) zur Abgabe einer Behandlungsdosis umfasst, das flexibel ist und das einen distalen Endabschnitt (10) aufweist, der zumindest über einen Teil seiner Länge in Längsrichtung in eine Vene (V) eingeführt werden kann und der es ermöglicht, dass mindestens im Bereich des Endes des distalen Endabschnitts (10) eine Behandlungsdosis in eine Vene (V) abgegeben wird, wobei die Vorrichtung zur endovenösen Behandlung ferner ein Antriebssystem (4) umfasst, das es ermöglicht, dass das faserförmige Element (1) zur Abgabe der Behandlungsdosis in mindestens einer ersten vorgegebenen Antriebsrichtung (R) angetrieben wird,
**dadurch gekennzeichnet, dass** sie ferner eine Führung (3) umfasst, die über die gesamte oder einen Teil ihrer Länge flexibel ist, die einen proximalen Endabschnitt (31) und einen distalen Endabschnitt (30) aufweist und die es ermöglicht, das faserförmige Element (1) zur Abgabe der Behandlungsdosis über einen Abschnitt seiner Länge mit einem proximalen Endabschnitt (11) zu führen, und andererseits ein distaler Endabschnitt (10) des faserförmigen Elements (1) zur Abgabe der Behandlungsdosis nicht von der Führung (3) geführt wird, und dadurch, dass sie ferner ein Haltesystem (5) umfasst, das den distalen Endabschnitt (30) der Führung (3) in Bezug auf den Körper eines Patienten in der Nähe der Einführzone (7) des faserförmigen Elements (1) zur Abgabe der Behandlungsdosis vorübergehend hält, wobei das faserförmige Element (1) zur Abgabe der Behandlungsdosis in seiner Längsrichtung in Bezug auf die Führung (3) verschiebbar ist, um zu ermöglichen, dass der distale Endabschnitt (10) des faserförmigen Elements (1) zur Abgabe der Behandlungsdosis in Längsrichtung über mindestens einen Abschnitt seiner Länge in eine Vene (V) eingeführt werden kann, wenn der distale Endabschnitt (30) der Führung (3) mittels des Haltesystems (5) relativ zum Körper eines Patienten gehalten wird, und Verriegelungsmittel (6), die den proximalen Endabschnitt (31) der Führung (3) zumindest in der Antriebsrichtung (R) des faserförmigen Elements (1) zur Abgabe der Behandlungsdosis axial verriegeln.

2. Vorrichtung nach Anspruch 1, wobei das Haltesystem (5) einen Halteteil (50; 50'; 50") umfasst, der dazu ausgelegt ist, am Körper eines Patienten angebracht zu werden, um den distalen Endabschnitt (30) der Führung (3) in Bezug auf den Körper eines Patienten vorübergehend in der Nähe der Einführzone (7) des faserförmigen Elements (1) zur Abgabe der Behandlungsdosis zu halten.

3. Vorrichtung nach Anspruch 2, wobei das Halteteil (50; 50'; 50") eine Unterseite (500a) zum Anbringen des Halteteils (50; 50'; 50") aufweist, die in Kontakt mit dem Körper eines Patienten steht, und wobei das faserförmige Element (1) zur Abgabe der Behandlungsdosis entlang seiner Länge verschiebbar ist, das durch den distalen Endabschnitt (30) der Führung (3) entlang einer Führungsachse (A) geführt ist, die die Unterseite (500a) des Halteteils (50 ; 50') nicht schneidet.

4. Vorrichtung nach Anspruch 3, wobei die Unterseite (500a) des Halteteils (50; 50'; 50") eine Stützfläche bildet und wobei das faserförmige Element (1) zur Abgabe der Behandlungsdosis aufgrund der Führung durch den proximalen Teil der Führung (3) entlang einer Führungsachse (A), die diese Stützfläche nicht schneidet, in seiner Längsrichtung verschiebbar ist.

5. Vorrichtung nach Anspruch 4, wobei die Unterseite (500a) des Halteteils (50; 50'; 50") eine ebene oder im Wesentlichen ebene Auflagefläche bildet oder so verformt werden kann, dass sie eine Auflagefläche bildet, die beim Aufbringen auf einen Körper eben oder im Wesentlichen eben ist, und die Führungsachse (A) im Wesentlichen parallel zu dieser Auflagefläche des Halteteils (50; 50') ist oder bei dem die Führungsachse (A) in Bezug auf die Auflagefläche des Halteteils (50") um einen Winkel (α) geneigt ist, der das Längseinführen des faserförmigen Elements (1) zur Abgabe der Behandlungsdosis in eine Vene ermöglicht, indem das faserförmige Element (1) zur Abgabe der Behandlungsdosis entlang seiner Längsrichtung verschoben wird, wenn die Auflagefläche auf den Körper aufgebracht wird.

6. Vorrichtung nach Anspruch 2, wobei der Halteteil eine Unterseite (500a) zum Aufbringen des Halteteils in Kontakt mit dem Körper eines Patienten aufweist, wobei die Unterseite (500a) des Halteteils eine Stützfläche bildet, die flach oder im Wesentlichen flach ist oder verformt werden kann, um eine Stützfläche zu bilden, die flach oder im Wesentlichen flach ist, wenn sie auf einen Körper aufgebracht wird, wobei das faserförmige Element (1) zur Abgabe der Behandlungsdosis in seiner Längsrichtung verschiebbar ist, durch Führen durch den distalen Endabschnitt (30) der Führung (3) entlang einer Führungsachse (A), die durch diese Lagerfläche verläuft und die in Bezug auf diese Lagerfläche um einen Winkel (α) geneigt ist, der das Längseinführen des faserförmigen Elements (1) zur Abgabe der Behandlungsdosis in eine Vene durch Verschieben des faserförmigen Elements (1) zur Abgabe der Behandlungsdosis entlang seiner Längsrichtung ermöglicht, wenn die Lagerfläche auf den Körper aufgebracht wird.

7. Vorrichtung nach einem der Ansprüche 5 oder 6, wobei der Winkel (α) kleiner oder gleich 60°, vorzugsweise kleiner oder gleich 45° und stärker bevorzugt kleiner oder gleich 30° ist.

8. Vorrichtung nach einem der Ansprüche 2 bis 7, wobei das Halteteil (50; 50'; 50") dauerhaft befestigt ist oder dauerhaft oder abnehmbar am distalen Endabschnitt (30) der Führung (3) befestigt werden kann.

9. Vorrichtung nach einem der vorhergehenden Ansprüche, ferner umfassend einen Einführkatheter (8), der vorübergehend mit dem distalen Endabschnitt (30) der Führung (3) verbunden werden kann.

10. Vorrichtung nach einem der vorhergehenden Ansprüche, wobei die Führung (3) so ausgebildet ist, dass nach dem Auffädeln auf das faserförmige Element (1) zur Abgabe der Behandlungsdosis in der Führung (3) ein Abschnitt (1c) des faserförmigen Elements (1) zur Abgabe der Behandlungsdosis verbleibt, der zugänglich ist und von einem Bediener bedient werden kann.

11. Vorrichtung nach einem der vorhergehenden Ansprüche, wobei das Haltesystem (5) Befestigungsmittel (51) umfasst, die es ermöglichen, den distalen Endabschnitt (30) der Führung (3) und gegebenenfalls das Halteteil (50; 50'; 50") zur Durchführung der Behandlung in der Nähe der Einführzone (7) des faserförmigen Elements (1) vorübergehend am Körper eines Patienten zu befestigen, und wobei vorzugsweise die Befestigungsmittel (51) einen Klebstoff oder eine Klebeschicht umfassen, die auf der Haut haften kann.

12. Vorrichtung gemäß einem der vorstehenden Ansprüche, wobei das Antriebssystem (4) ein Entnahmesystem ist, das das faserförmige Element (1) zur Abgabe der Behandlungsdosis in einer Antriebsrichtung (R) rückwärts antreibt, um das Entfernen des distalen Endabschnitts (10) des faserförmigen Elements (1) zur Abgabe der Behandlungsdosis aus einer Vene zu ermöglichen.

13. Vorrichtung nach einem der vorhergehenden Ansprüche, bei der die Verriegelungsmittel (6) dazu ausgelegt sind, den proximalen Endabschnitt (31) der Führung (3) auch in der der Antriebsrichtung (R) des faserförmigen Elements (1) zur Abgabe der Behandlungsdosis entgegengesetzten Richtung (F) axial zu verriegeln.

14. Vorrichtung nach einem der vorhergehenden Ansprüche, wobei das faserförmige Element (1) zur Abgabe einer Behandlungsdosis eine optische Faser ist, vorzugsweise ferner eine Quelle elektromagnetischer Strahlung (2) umfassend, die mit der optischen Faser (1) gekoppelt werden kann.

15. Vorrichtung nach einem der vorhergehenden Ansprüche, wobei die Verriegelungsmittel (6) einen zweiteiligen Verbinder umfassen, einen festen Teil (60A; 61A), der am Antriebssystem (4) befestigt ist, und einen abnehmbaren Teil (60B; 61B), der am proximalen Endabschnitt (31) der Führung (3) befestigt ist und mit dem festen Teil (60A; 61A) zum Verriegeln des proximalen Endabschnitts (31) der Führung (3) zusammenwirken kann.

16. Vorrichtung nach einem der vorhergehenden Ansprüche, wobei die Führung (3) eine flexible Führungshülle (3A) oder mehrere zusammengesetzte Führungselemente umfasst, die mindestens ein flexibles Führungselement (3A) und insbesondere eine flexible Führungshülle (3A) umfassen.

## Claims

1. An endovenous treatment device having a delivery system (1 ; 2) of at least one treatment dose, which delivery system (1 ; 2) comprises a wired treatment dose delivery element (1) which is flexible, which has a distal end portion (10) capable of being inserted, at least over part of its length, longitudinally into a vein (V), and which enables delivery into a vein (V) of at least one treatment dose in the region of the end of said distal end portion (10), the endovenous treatment device further comprising a drive system (4) for driving of the wired treatment dose delivery element (1) in at least a given first drive direction (R), **characterized in that** it further comprises a guide (3) which is flexible over all or part of its length, which has a proximal end portion (31) and a distal end portion (30), and which allows the wired treatment dose delivery element (1) to be guided over a portion of its length with a proximal end portion (11) and, at the opposite end, a distal end portion (10) of the wired treatment dose delivery element (1), which are not guided by the guide (3), and **in that** it further comprises a holding system (5) which enables the distal end portion (30) of the guide (3) to be temporarily held relative to the body of a patient, near the insertion zone (7) of the wired treatment dose delivery element (1), the wired treatment dose delivery element (1) being slidable in the direction of its length relative to the guide (3), so as to allow insertion longitudinally into a vein (V) of the distal end portion (10) of the wired treatment dose delivery element (1) over at least a portion of its length when the distal end portion (30) of the guide (3) is held relative to the body of a patient by means of the holding system (5), and locking means (6), which allow axially the proximal end portion (31) of the guide (3) to be locked, at least in the drive direction (R) of the wired element (1) for treatment dose delivery.

2. A device according to claim 1, wherein the holding system (5) comprises a holding part (50 ; 50'; 50'ʹ) which is designed to be applied to the body of a patient so as to temporarily maintain the distal end portion (30) of the guide (3) relative to the body of a patient, near the insertion zone (7) of the wired treatment dose delivery element (1).

3. Device according to claim 2, wherein the holding part (50 ; 50' ; 50") has an underside (500a) for applying the holding part (50 ; 50'; 50") in contact with the body of a patient, and wherein the wired treatment dose delivery element (1) is slidable along the direction of its length, guided by the distal end portion (30) of the guide (3) along a guide axis (A) which does not intersect the lower face (500a) of the holding part (50 ; 50').

4. Device according to claim 3, wherein the underside (500a) of the holding part (50 ; 50' ; 50") forms a support surface, and wherein the wired treatment dose delivery element (1) is slidable in the direction of its length being guided by the proximal portion of the guide (3), along a guide axis (A) which does not intersect the support surface.

5. Device according to claim 4, wherein the underside (500a) of the holding part (50 ; 50' ; 50") forms a support surface which is flat or substantially flat, or is capable of being deformed so as to form a support surface which is flat or substantially flat when applied to a body, and the guide axis (A) is substantially parallel to this support surface of the holding part (50; 50') ou wherein the guide axis (A) is inclined relative to the support surface of the holding part (50") at an angle (α), which allows the insertion longitudinally of the wired vein treatment dose delivery element (1), by sliding the wired element (1) for delivery treatment dose in the direction of its length, when the support surface is applied against the body.

6. Device according to claim 2, wherein the holding part has a lower face (500a) for applying the holding part in contact with the body of a patient, wherein the lower face (500a) of the holding part forms a support surface which is flat or substantially flat, or is capable of being deformed so as to form a support surface which is flat or substantially flat when it is applied to a body, in which the wired treatment dose delivery element (1) is capable of sliding in the direction of its length, being guided by the distal end portion (30) of the guide (3) along a guide axis (A), which passes through this support surface and which is inclined with respect to this support surface by an angle (α) permitting longitudinal insertion of the wired treatment dose delivery element (1) into a vein, by sliding of the wired treatment dose delivery element (1) in the direction of its length, when the support surface is applied against the body.

7. Device according to any one of claims 5 or 6, wherein said angle (α) is less than or equal to 60°, preferably less than or equal to 45°, and more preferably less than or equal to 30°.

8. Device according to any one of claims 2 to 7, wherein the holding part (50 ; 50'; 50") is permanently attached or can be permanently or removably attached to the distal end portion (30) of the guide (3).

9. Device according to anyone of the preceding claims, further comprising an introducer catheter (8) which is designed to be temporarily assembled with the distal end portion (30) of the guide (3).

10. Device according to any one of the preceding claims, wherein the guide (3) is designed such that once slid onto the wired treatment dose delivery element (1), there remains in the guide (3) a portion (1c) of the wired treatment dose delivery element (1) that is accessible and can be manipulated by an operator.

11. Device according to any one of the preceding claims, wherein the holding system (5) comprises attachment facilities (51) which temporarily attach the distal end portion (30) of the guide (3), and, if appropriate, the holding part (50 ; 50' ; 50") onto the body of a patient near the insertion area (7) of the wired treatment dose delivery element (1) and preferably wherein the attachment facility (51) comprises an adhesive or an adhesive layer capable of bonding to the skin.

12. Device according to any one of the preceding claims, wherein the drive system (4) is a retraction system which drives the wired treatment dose delivery element (1) rearward in the drive direction (R) allowing the removal from a vein of the distal end portion (10) of the wired treatment dose delivery element (1).

13. Device according to any one of the preceding claims, wherein the locking means (6) are also designed to axially lock the proximal end portion (31) of the guide (3) in the direction (F) opposite to the drive direction (R) of the wired treatment dose delivery element (1).

14. Device according to any one of the preceding claims, wherein the wired treatment dose delivery element (1) is an optical fiber and preferably comprising a source of electromagnetic radiation (2) which can be coupled to the optical fiber (1).

15. Device according to any of the preceding claims, wherein the locking means (6) comprise a two-part connector, a fixed part (60A; 61A) which is attached on the drive system (4), and a removable part (60B; 61B) which is attached to the proximal end portion (31) of the guide (3) and is designed to interoperate with the attached portion (60A; 61A) for locking of the proximal end portion (31) of the guide (3).

16. Device according to any one of the preceding claims, wherein the guide (3) comprises a flexible guide sheath (3A) or comprises several assembled guide elements, including at least one flexible guiding element (3A), and more particularly a flexible guide sheath (3A).
